(19) Europäisches Patentamt
European Patent Office
Office européen des brevets

(11) Numéro de publication: **0 000 315**
**B1**

(12) # FASCICULE DE BREVET EUROPEEN

(21) Numéro de dépôt: **78420001.6**

(22) Date de dépôt: **14.06.78**

(51) Int. Cl.³: **C 07 C 47/20,**
C 07 C 45/00, C 07 B 1/00,
B 01 J 31/24

(54) **Procédé de préparation de citronellal optiquement actif**

(30) Priorité: **04.07.77 FR 7721377**

(43) Date de publication de la demande:
**10.01.79 Bulletin 79/01**

(45) Mention de la délivrance du brevet:
**17.09.80 Bulletin 80/19**

(84) Etats contractants désignés:
**BE CH DE GB NL**

(56) Documents cités:
**DE - A - 2 161 200**
**FR - A - 2 314 911**
**US - A - 3 849 480**
**US - A - 3 939 188**

(73) Titulaire: **RHONE-POULENC INDUSTRIES 22, avenue Montaigne**
**F - 75008 Paris (FR)**

(72) Inventeur: **Aviron-Violet, Paul**
**25 bis, chemin de la Citadelle**
**F - 69230 St-Genis Laval (FR)**
**Dang, Tuan-Phat**
**29, rue du Commandant Faurax**
**F - 69006 Lyon (FR)**

(74) Mandataire: **Rioufrays, Roger**
**RHONE-POULENC INDUSTRIES**
**Centre de Recherches**
**des Carrières Service Brevets**
**F - 69190 Saint-Fons (FR)**

**EP 0 000 315 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de Préparation de citronellal optiquement actif

La présente invention a pour objet un procéde de préparation de citronellal optiquement actif, encore dénommé citronellal chiral, par hydrogénation asymétrique du néral (E-diméthyl-3,7 octadiène-2,6 al) ou du géranial (Z-diméthyl-3,7 octadiène-2,6 al) isomères achiraux constitutifs du citral.

Le citronellal chiral est un intermédiaire apprécié en synthèse organique; en particulier le d-citronellal est utilisé pour la préparation du (—) (1S) menthol désigné ci-après 1-menthol, par un procédé qui met en oeuvre la cyclisation du d-citronellal en (—) (1S)-isopulégol sous l'influence d'un catalyseur protonique ou par voie thermique, puis l'hydrogénation de l'isopulégol en (1)-menthol (cf. J.C. LEFFINGWELL et R.E. SHACKELFORD, Cosmetics and Perfumery *89* 70—78/1974/).

Le d-citronellal utilisé pour la synthèse du 1-menthol est obtenu à partir de diverses essences naturelles contenant principalement du d-citronellal et en particulier à partir de l'essence de citronelle. L'emploi de citronellal chiral d'origine naturelle n'est pas entièrement satisfaisant dans la mesure où, en raison de la fluctuation des prix des produits naturels, il arrive périodiquement que le prix du d-citronellal soit plus élevé que celui du menthol naturel. Il importe donc à l'industrie de disposer d'une source de citronellal chiral ayant un prix relativement stable et conduisant à un 1-menthol synthétique dont le prix de revient soit inférieur à celui du menthol naturel.

Il existe divers procédés d'hydrogénation du citral en citronellal racémique. Ainsi dans la demande de brevet français No 75-19.070, publiée sous le No 2.314.911, on a décrit un procédé d'hydrogénation sélective en phase hétérogène du citral en citronellal en présence d'un catalyseur au palladium métallique déposé sur un support inerte et d'un agent alcalin (hydroxyde alcalin ou alcalino-terreux) en milieu hydroalcoolique. Bien que ce procédé permette une hydrogénation sélective de la double liaison en $\alpha$ du groupe carbonyle, il ne peut conduire à l'obtention de citronellal optiquement actif.

On connaît divers complexes achiraux du rhodium avec des ligands du type des trialcoyl- ou triarylphosphines et leur emploi comme catalyseurs d'hydrofromylation des oléfines et d'hydroaldolisation des aldéhydes (cf. brevet américain No 3.939.188). On a bien proposé dans le brevet des Etats-Unis d'Amérique No 3.849.480 un procédé d'hydrogènation de composés à insaturation éthyléniques tels que l'acide $\alpha$-phénylacrylique, la méthyl-3 cyclohexène-2 one et l'acide méthyl-2 butène-2 oïque, mais aucun de ces composés ne comporte une double insaturation éthylénique liée à deux atomes de carbone asymétriques de sorte que dans ces cas là, il n'y a pas de risque de formation d'isomères. L'art antérieur a enseigné divers types de catalyseurs pouvant convenir à la réalisation de synthèses asymétriques. C'est ainsi qu'outre le brevet américain No 3.849.480 précité, la demande allemande No 2.161.200 décrit des complexes du rhodium avec des ligands optiquement actifs particuliers du groupe des diphosphines, des diarsines ou des distibines et enseigne leur emploi en synthèse asymétrique, en particulier pour l'hydrogénation de l'acide $\alpha$-acétylaminocinnamique en acétylalanine. Cependant jusqu'ici il n'a jamais été proposé de préparer le citronellal chiral par hydrogénation asymétrique du citral.

Le citronellal synthétique achiral obtenu par hydrogénation du citral (mélange de néral et de géranial) pourrait constituer une source intéressante de citronellal chiral et notamment de d-citronellal, toutefois il n'existe pas de procédé industriel de dédoublement du citronellal racémique en ses énantiomères, de sorte que l'industrie ne dispose d'aucun procédé permettant d'obtenir des citronellals chiraux par voie synthétique. La présente invention se propose précisément de résoudre un tel problème.

Plus particulièrement la présente invention a pour objet un procédé de préparation de citronellal optiquement actif caractérisé en ce que l'on hydrogène le néral ou le géranial en présence d'un catalyseur constitué par un complexe soluble dans le milieu réactionnel formé à partir d'un dérivé du rhodium et d'une phosphine chirale.

Par phosphine chirale on désigne une phosphine ou diphosphine dont l'un au moins des restes organiques liés à l'atome de phosphore comporte au moins un atome de carbone chiral, et/ou dont un au moins des atomes de phosphore est chiral.

Le complexe soluble du dérivé du rhodium et de la phosphine chirale peut être préparé extemporanément ou être formé "in situ" dans les conditions de la réaction par mise en oeuvre des constituants du complexe. Cette dernière façon de faire qui a l'avantage de la simplicité est généralement préférée.

Comme dérivés du rhodium convenant à la mise en oeuvre du procédé selon l'invention on utilise des dérivés du rhodium comportant des restes de nature diverses. Il peut s'agir de sels de rhodium d'acides minéraux ou organiques ou de complexes du rhodium dont les ligands peuvent être remplacés par la phosphine chirale. On peut par exemple faire appel à des halogénures de rhodium tel que le trichlorure de rhodium hydraté; à des complexes du rhodium avec les oléfines de formule générale:

$$[Rh\ X\ (L)_x]\ 2 \qquad (I)$$

dans laquelle X représente un atome d'halogène: chlore ou brome par exemple, x est un nombre entier de 1 à 4 et L une oléfine ou une dioléfine aliphatique ou cycloaliphatique telle que l'éthylène, le propylène, le butène, l'isobutène, le butadiène, l'hexadiène-1,5, l'heptadiène-1,4, l'octadiène-1,5,

l'isoprène le cyclohexadiène-1,3,le cyclooctadiene-1,5; comme exemples de tels complexes ont peut citer le $\mu,\mu'$ dichloro bis-(cyclohexadiène-1,3-rhodium), le $\mu,\mu'$-dichloro bis-(cyclooctadiène-1,5 rhodium), le $\mu,\mu'$-chloro bis(diethylène rhodium)); on peut encore faire appel à des complexes rhodium carbonyle et à leurs dérivés tels que ceux répondant à la formule générale:

$$Rh\ H(CO)\ (L_1)_3 \qquad (II)$$

dans laquelle $L_1$ représente un ligand mono- ou polydentate et notamment une phophine achirale de formule générale:

$$P\ (R)_3$$

dans laquelle R représente un radical achiral alcoyle, cycloalcoyle aryle ayanat de 1 à 10 atomes de carbone tel que les radicaux méthyle, éthyle, propyle, butyles, pentyles, hexyles, octyles, cyclohexyle, phényle, toluyle. De préférence $L_1$ est la triphénylphosphine. Parmi les dérivés du rhodium précités on fait appel de préférence aux divers rhodiumcarbonyle et notamment au tétrarhodium dodécacarbonyle et à l'hexarhodiumhexadécacarbonyle. Les complexes du rhodium pris sous forme cationique peuvent également être utilisés.

Comme phosphine chirale convenant à la mise en oeuvre de l'invention on peut utiliser aussi bien des monophosphines que des diphosphines. Comme exemples de monophosphines chirale on peut citer la diphénylmenthylphosphine, la phényldimenthylphosphine et la trimenthylphosphine. On préfére toutefois faire appel à des diphosphines chirales de formule générale:

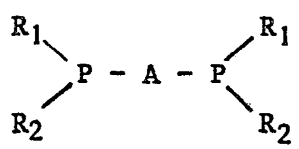

dans laquelle:

— $R_1$ et $R_2$, identiques ou différents, représentent des radicaux hydrocarbonés ayant de 1 à 15 atomes de carbone,

— A représente un lien valentiel ou un radical organique divalent éventuellement substitué par un ou plusieurs groupes fonctionnels inertes,

l'un au moins des radicaux $R_1$, $R_2$ et A étant chiral.

Plus spécifiquement $R_1$ et $R_2$, qui sont de préférence identiques, représentent des radicaux alcoyles ayant de 1 à 10 atomes de carbone (méthyle, éthyle, isobutyle, sec-butyle, sec-pentyle, éthyl-2 hexyle), cycloalcoyle ayant de 4 à 8 atomes de carbone cycliques (cyclobutyle, méthyle-1 cyclobutyle, cyclohexyle, méthyle-1 cyclohexyle, méthyle-2 cyclohexyle), aryles ou alcoylaryles (phényle, naphthyle, toluyle). A représente:

— un radical alcoylène linéaire ou ramifié ayant de 1 à 10 atomes de carbone, un radical cycloalcoylène ayant de 3 à 7 atomes de carbone cycliques, éventuellement susbtitué par 1 à 3 groupes alcoyles ayant de 1 à 4 atomes de carbone, un radical arylène, un radical polycyclique divalent, ces radicaux étant éventuellement substitués par 1 ou plusieurs groupes fonctionnels inertes et notamment 1 à 3 groupes alcoxy ayant de 1 à 4 atomes de carbone.

— un groupe hétérocyclique divalent (pyridylène, dioxa-1,3 cyclopentylène-4,5) ayant 1 ou 2 hétéroatomes tels que l'oxygène et/ou l'azote.

— un enchaînement de 1 ou plusieurs radicaux alcoylène et/ou cycloalcoylènes et/ou hétérocycliques et/ou polycycliques divalents tel que ceux définis précédemment.

— un enchaînement de groupes alcoylènes tels que ceux définis précédemment et de groupes amino tertiaire qui peuvent éventuellement être liés directement aux atomes de phosphore par l'intermédiaire de l'atome d'azote.

Comme exemple de radicaux alcoylènes ont peut citer les radicaux méthylène, éthylène, propylène, éthyl-2 propylène; A peut encore représenter un radical cyclobutylène; cyclohexylène-1,4; méthyl-2 cyclohexylène-1,4; un radical ortho- ou p-phénylène; un radical diméthoxy-2,3 butylène-1,4.

Comme exemple de radicaux A chiraux divalents formés par un enchaînement de radicaux alcoylènes et cycloalcoylènes ou hétérocycliques ou polycycliques ou amino on peut citer ceux de formules:

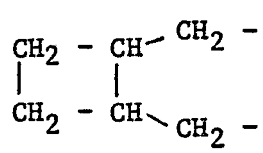

(a)

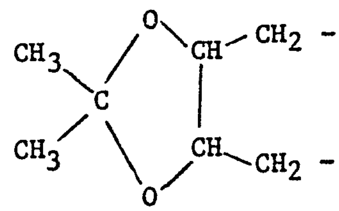

(b)

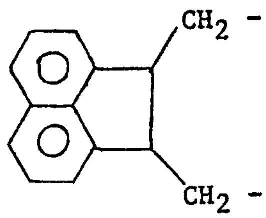

(c)

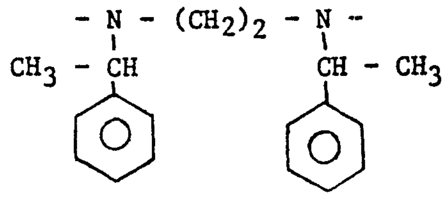

(d)

Parmi les diphosphines chirales qui peuvent être utilisées dans le procédé selon l'invention on peut citer à titre non limitatif: le bis(diphénylphosphinométhyl)-1,2-cyclobutane (DPCB), le bis(diméthylphophinométhyl)-1,2 cyclobutane, le bis(di-n-butylphosphinométhyl)-1,2 cyclobutane, le bis(dioctylphosphinométhyl-)-1,2 cyclobutane, le bis(ditolylphosphinométhyl-1,2 cyclobutane, le bis(dinaphtylphosphinométhyl)-1,2 cyclobutane, le bis(éthyl, hexylphosphinométhyl)-1,2 cyclobutane, le bis(diphénylphosphinométhyl)-1,2 cyclopentane; le bis(diphénylphosphinométhyl)-1,2 cyclohexane; le bis-(diméthylphosphinométhyl)-4,5 diméthyl-2,2 dioxolane-1,3, le bis-(diphénylphosphinométhyl)-4,5 diméthyl-2,2 dioxolane-1,3, (DIOP), le bis(ditolylphosphinométhyl)-4,5 diméthyl-2,2 dioxolane-1,3 le bis(diméthylphosphinométhyl)-1,2 acénaphtène, le bis(dibutylphosphinométhyl)-1,2 acénaphtène, le bis-(diphénylphosphinométhyl)-1,2 acénaphtène (DPA), le bis(ditolylphosphinométhyl)-1,2 acénaphtène, le bis(diphénylphosphino)-1,4 diméthoxy-2,3 butane (DDB), la tétramenthyldiphosphine; le bis (N,N'-diphénylphosphino)bis(N,N'(phényl-1 éthyl))diaza-1,4 butane.

Parmi les phosphines citées précédemment on utilise de préférence les bis(diarylphosphinométhyl)-1,2 cyclobutanes décrits dans le brevet français no 73/18 319.

Comme exemples de phosphines à atomes de phosphore chiral on peut citer la méthyl cyclohexyl orthométhoxyphényl phosphine; la méthylcyclohexylphénylphosphine; la benzylphénylméthylphosphine.

Les complexes dérivés de $Rh_4\ (CO)_{12}$ ou de $Rh_6\ (CO)_{16}$ et des bis(diarylphosphinométhyl)-1,2 cyclobutanes conviennent tout particulièrement bien à l'hydrogenation asymétrique du néral et du géranial en énantiomères du citronellal car ils procurent à la fois une vitesse élevée d'hydrogénation, une bonne sélectivité en citronellal et une bonne pureté optique.

La quantité de dérivé du rhodium mise en oeuvre dans le procédé de l'invention, exprimée en atomes-grammes de métal par mole d'aldéhyde diénique à hydrogéner peut varier dans de larges limites. Qu'il s'agisse du complexe préformé ou du dérivé apte à engendrer ce complexe dans les conditions de la réaction la quantité peut être choisie pour que le nombre d'atomes-grammes de rhodium par mole d'aldéhyde soit compris entre $1 \times 10^{-4}$ et $1 \times 10^{-1}$.

Lorsque le complexe dérivé du rhodium/phosphine chirale est préparé "in situ", la quantité de phosphine engagée dans le processus dépend de la nature de la phosphine et de celle du dérivé du rhodium. Cette quantité, exprimée par le nombre d'atomes-grammes de phophore par atome-gramme de rhodium est telle que ce rapport peut varier entre 0,5 et 10; de préferénce le rapport P/Rh est compris entre 1 et 6. On pourrait cependant mettre en oeuvre des rapports P/Rh supérieurs à 10 sans sortir du cadre de la présente invention, mais celà ne procurerait aucun avantage particulier.

La température à laquelle on conduit l'hydrogénation n'est pas critique et peut varier dans le larges limites. En général elle est comprise entre 0 et 150°C et de préférence entre 10 et 100°C. Il en est de même de la pression d'hydrogène qui peut varier entre 0,1 et 100 bars et de préférence entre 0,5 et 50 bars.

Bien qu'il soit préférable de soumettre à l'hydrogénation asymétrique un aldéhyde diénique aussi pur que possible, c'est-à-dire pratiquement exempt de son isomère, on peut mettre en oeuvre du néral contenant jusqu'à 15% de géranial et vice versa.

De la même façon il est préférable d'utiliser une phosphine chirale ne contenant pas son énantiomère bien que l'on puisse opérer avec une phosphine chirale contenant moins de 15% de son énantiomère.

L'hydrogénation asymétrique du néral ou du géranial est de préférence conduite dans un solvant inerte de l'aldéhyde et du catalyseur. Comme exemple de solvants, on peut citer des hydrocarbures (hexane, heptane, cyclohexane, benzène, toluène), des alcools (méthanol, éthanol), des nitriles (acétonitrile, benzonitrile).

Les exemples suivants illustrent l'invention et montrent comment elle peut être mise en pratique. Dans ces exemples on désignera par pureté optique P.O le rapport du pouvoir rotatoire $(\alpha_1)_D$ du produit obtenu par le procédé au pouvoir rotatoire $(\alpha)_D$ du produit mesuré dans les mêmes conditions, multiplié par 100, soit

$$\text{P.O en \%} = \frac{(\alpha_1)_D}{(\alpha)_D} 100$$

Par rendement optique on désigne la valeur de la pureté optique du produit que l'on obtiendrait par utilisation d'une phosphine optiquement pure.

EXEMPLE 1

Dans un ballon en verre de 50 $cm^3$, équipé d'une arrivée de gaz par tube plongeant, d'un thermomètre, d'une agitation magnétique et d'un bouchon en verre permettant de procéder à des additions de réactifs ou des prélèvements de masse réactionnelle au moyen d'une seringue, on charge 18,2 mg de $Rh_6(CO)_{16}$ (1,02 × $10^{-4}$ at-g de Rh), 67,5 mg (+)-(DPCB) soit 0,15 millimole, puis on purge l'appareil à l'azote et injecte 20 $cm^3$ de toluène. On agite le contenu du ballon pendant 1 heure sous atmosphère d'azote puis ajoute 1,79 g (soit 11,77 millimole) de géranial contenant 5% de néral. On purge l'appareil à l'hydrogène puis maintient le contenu du ballon sous 1 bar d'hydrogène pendant 4 heures à 25°C. La réaction est arrêtée et la masse réactionnelle est soumise à une analyse chromatographique en phase gazeuse: le taux de transformation du géranial est de 100% et le rendement en citronellal de 99%. On évapore le solvant puis distille le résidu sous pression réduite. On recueille ainsi 1,28 g de 1-citronellal ayant un pouvoir rotatoire $(\alpha)_D^{25} = -8{,}76°$ (mesuré sur une solution à 6 g pour 100 $cm^3$ dans l'hexane) et $(\alpha)_D^{25} = -9{,}1°$ mesuré en absence de solvant. Le pouvoir rotatoire du 1-citronellal pur $(\alpha)_D^{25}$ mesuré sur une solution à 6 g/100 $cm^3$ dans le cyclohexane est de −15,6°. Par référence à cette valeur, la P.O du produit obtenu est de 56%. Le pouvoir rotatoire du 1-citronellal pur (détermination sans solvant) est $(\alpha)_D^{25} = -16°$ (cf. DONELL et al. Australian J. Chem. *19* 525 [1966]).

EXEMPLE 2

On opère comme à l'exemple 1 sur les quantités suivantes:

| | |
|---|---|
| — $Rh_6(CO)_{16}$ | 17,8 mg |
| — (+)-DPCB | 68 mg |
| — néral à 12% de géranial | 11 g |

La durée de réaction est de 10 heures

| | |
|---|---|
| — taux de transformation | 100% |
| — rendement en citronellal | 99% |

Après distillation on recueille 9,6 g citronellal de $(\alpha)_D^{25} = +10{,}15°$ (solution à 6 g/100 $cm^3$ dans l'hexane) soit une pureté optique de 65%.

EXEMPLES 3 A 4

On opère comme à l'exemple 1 en remplaçant la (+)-DPCB par la (+)-DIOP. Le rapport géranial/Rh est de 120 et on utilise successivement un rapport P/Rh de 4 et de 6.

Dans ces conditions on a obtenu les résultats suivants:

| Exemples | P/Rh | Durée heures | Taux de transformation | RT (1) % | PO | $(\alpha)_D^{25}$ |
|---|---|---|---|---|---|---|
| 3 | 4 | 20 | 97 | 98 | 51 | + 8,2° (2) |
| 4 | 6 | 20 | 98 | 99 | 5P | + 8° (2) |

(1) rendement en citronellal par rapport au géranial transformé

(2) mesuré sur le produit pur.

## EXEMPLE 5

On opère comme à l'exemple 1 en remplaçant la (+)-DPCB par la (—)-DPCB. Le rapport du nombre de mole de géranial (G) au nombre d'atomes-grammes de rhodium (G/Rh) est égal à 123 et le rapport P/Rh à 4. La (—)-DPCB a une pureté optique de 95,5%.

La durée de réaction est de 18 heures, le taux de transformation du géranial de 99%, le RT en citronellal de 99%. La pureté optique du (d)-citronellal obtenu est de 49% [$(\alpha)_D^{25} = +7,7°$: solution à 6 g/100 $cm^3$ dans l'hexane]. Si l'on tient compte de la pureté de la phosphine le rendement optique s'élève à 52%.

## EXEMPLE 6

On opère comme à l'exemple 5 en remplaçant le géranial par le néral, les autres conditions étant par ailleurs identiques. On a obtenu les résultats suivants:

| | |
|---|---|
| — durée d'hydrogénation | 18 heures |
| — taux de transformation du néral | 100% |
| — RT en citronellal | 88% |
| — $[\alpha]_D^{25}$ à 6 g/100 $cm^3$ dans l'hexane. | —9° |
| — pureté optique | 57% |

## EXEMPLE 7

On opère suivant le mode opératoire de l'exemple 1 et dans les mêmes conditions de pression et de température en remplaçant ($Rh_6(CO)_{16}$ par $Rh_4(CO)_{12}$. Le rapport P/Rh est de 3 et le rapport G/Rh de 120. Les résultats sont les suivants:

| | |
|---|---|
| — durée | 3 h 30 mn |
| — TT | 100% |
| — RT en citronellal | 99% |
| — $(\alpha)_D^{25}$ dans l'hexane | —8,1° |
| — P.O | 52% |

## EXEMPLE 8

On opère comme à l'exemple 7 en remplaçant le géranial par le néral (le rapport N/Rh est de 140). On a obtenu les résultats suivants:

| | |
|---|---|
| — durée | 2 h 45 mn |
| — TT du néral | 100% |
| — RT en citronellal | 100% |
| — $(\alpha)_D^{25}$ mesuré dans l'hexane | +10,3° |
| — P.O. | 66% |

EXEMPLE 9

On opère comme à l'exemple 1 en portant le rapport N/Rh à 750 au lieu de 115 et le rapport P/Rh à 2. On a obtenu les résultats suivants:

| | |
|---|---|
| — durée | 6 h 40 mn |
| — TT du néral | 100% |
| — RT en citronellal | 100% |
| — $(\alpha)_D^{25}$ mesurée dans l'hexane | +10,9° |
| — P.O. | 70% |

Le néral utilisé contenait 7% de géranial.

EXEMPLES 10 à 18

On opère selon le mode opératoire et les conditions de température et de pression de l'exemple 1, en faisant varier la nature de l'aldéhyde, de la phosphine chirale et du dérivé du rhodium. Les autres conditions et les résultats obtenus figurent dans le tableau suivant:

| Exemples | Phosphine | DERIVE DU RHODIUM | ENAL | P/Rh | ENAL/Rh | Durée en h | TT % ENAL | RT % | $(\alpha)_D^{25}$ | PO % |
|---|---|---|---|---|---|---|---|---|---|---|
| 10 | (+)–DPCB | $RhH(CO)(P\emptyset_3)_3$ (1) | géranial | 6 | 123 | 70 | 81 | 99 | –7,6 (2) | 58 |
| 11 | (–)–DIOP | ,, | ,, | 6 | 134 | 108 | 83 | 100 | +7,3 (3) | 54 |
| 12 | (–)–DPCB | ,, | ,, | 2 | 149 | 29 | 95 | 100 | +8,7 (3) | 54 |
| 13 | ,, | ,, | néral | 6 | 108 | 73 | 93 | 98 | –10 (3) | 67 |
| 14 | ,, | ,, | géranial | 2 | 570 | 40 | 96 | 100 | +8,4 (3) | 55 |
| 15 | ,, | ,, | ,, | 2 | 2100 | 65 | 95 | 98 | +9 (2) | 58 |
| 16 | (–)–DDB | ,, | ,, | 2,6 | 114 | 28 | 68 | 98 | +1,9 (3) | 19,5 |
| 17 | ,, | ,, | néral | 2 | 105 | 65 | 67 | 98 | –2,1 (3) | 20 |
| 18 | (+)–DPA | ,, | géranial | 2 | 136 | 22 | 40 | 98 | –1,7 (3) | 27 |

(1) dans la formule $RhH(CO)(P\emptyset_3)_3$ $P\emptyset_3$ désigne la triphénylphosphine

(2) mesuré sur une solution 6 g/100 cm3 dans l'hexane

(3) mesuré sur le produit obtenu

EXEMPLE 19

Dans un autoclave en acier inoxydable de 125 $cm^3$ équipé d'un système d'agitation à secousses, on introduit une ampoule de verre de 35 $cm^3$ contenant 10 $cm^3$ de toluène, 18,3 mg de (—)-DPCB, 91,9 mg de RhH(CO) $(PØ)_3$ et 1,91 g de géranial. On ferme l'autoclave et introduit de l'hydrogène jusqu'à une pression de 25 bars. On maintient 17 heures dans ces conditions, puis l'autoclave est dégazé et le contenu de l'ampoule est traité et analysé comme à l'exemple 1.

Le taux de transformation du géranial s'élève à 69% le rendement en citronellal par rapport au géranial transformé à 99% et la pureté optique à 60% (pouvoir rotatoire $(\alpha)_D^{25} = +6{,}6°$ mesuré sur le produit pur).

**Revendications**

1. Procédé de préparation de citronellal optiquement actif, caractérisé en ce que l'on hydrogène le néral ou de géranial en présence d'un catalyseur constitué par un complexe soluble dans le milieu réactionnel, formé à partir d'un dérivé du rhodium et d'une phosphine chirale.

2. Procédé selon la revendication 1, caractérisé en ce que le complexe du rhodium et de la phosphine chirale est préparé extemporanément.

3. Procédé selon la revendication 1, caractérisé en ce que le complexe du rhodium et de la phosphine chirale est formé "in situ" à partir d'un dérivé du rhodium et d'une phosphine chirale.

4. Procédé selon la revendication 3, caractérisé en ce que le dérivé rhodium est un sel d'acide minéral ou organique ou un complexe du rhodium avec ligand achiral.

5. Procédé selon la revendication 4, caractérisé en ce que le dérivé du rhodium est le trichlorure de rhodium.

6. Procédé selon la revendication 4, caractérisé en ce que le complexe du rhodium a pour formule générale:

$$[Rh\ X\ (L)_x]_2 \qquad (I)$$

dans laquelle:

— X représente un atome d'halogène

— x est un nombre entier de 1 à 4

— L représente une mono- ou dioléfine.

7. Procédé selon la revendication 6, caractérisé en ce que le complexe du rhodium est le $\mu,\mu'$-dichloro bis(cyclooctadiène-1,5 rhodium).

8. Procédé selon la revendication 4, caractérise en ce que le complexe du rhodium a la formule générale:

$$Rh\ H\ (CO)\ (PR_3)_3 \qquad (II)$$

dans laquelle R représente un radical alcoyle, cycloalcoyle ou aryle achiral ayant de 1 à 10 atomes de carbone.

9. Procédé selon la revendication 8, caractérisé en ce que le complexe du rhodium a la formule Rh H $(CO)[P—(C_6H_5)_3]_3$.

10. Procédé selon la revendication 4, caractérisé en ce que le complexe du rhodium est un rhodium carbonyle pris dans le groupe du tétrarhodiumdodécacarbonyle et de l'hexarhodium-hexadécarbonyle.

11. Procédé selon l'une quelconque des revendications 1 à 10, caractérisé en ce que la phosphine chirale comporte au moins un atome de carbone chiral et/ou au moins un atome de phosphore chiral.

12. Procédé selon la revendication 11, caractérisé en ce que la phosphine chirale est une disphosphine de formule générale:

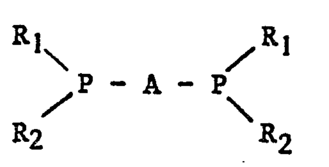

(III)

dans laquelle:

— $R_1$ et $R_2$, identiques ou différents, représentent des radicaux hydrocarbonés ayant de 1 à 15 atomes de carbone,

— A représente un lien valentiel ou un radical organique divalent éventuellement substitué par un ou plusieurs groupes fonctionnels inertes, l'un au moins des radicaux $R_1$, $R_2$ et A étant chiral.

13. Procédé selon de revendication 12, caractérisé en ce que l'on utilise une diphosphine chirale de formule (III) dans laquelle $R_1$ et $R_2$ représentent des radicaux alcoyles ayant de 1 à 10 atomes de carbone, cycloalcoyles ayant de 4 à 8 atomes de carbone cycliques, aryles ou alcoylaryles et A symbolise:

a) un radical alcoylène linéaire ou ramifié ayant de 1 à 10 atomes de carbone, un radical cycloalcoylène ayant de 3 à 7 atomes de carbone cycliques éventuellement substitué par 1 à 3 radicaux alcoyles ayant de 1 à 4 atomes de carbone, un radical arylène, un radical polycyclique divalent, lesdits radicaux pouvant être substitué par un ou plusieurs groupes alcoxy ayant de 1 à 4 atomes de carbone.

b) un groupe hétérocyclique divalent ayant 1 ou 2 hétéroatomes du groupe de l'oxygène et de l'azote.

c) un enchaînement de 1 ou plusieurs radicaux alcoylènes et/ou cycloalcoylènes et/ou hétérocycliques et/ou polycycliques divalents tels que ceux définis précédemment sous a) et b).

d) un enchaînement de groupes alcoylènes tels que ceux définis précédemment sous a) et de groupes amino tertiaires qui peuvent être liés directement aux atomes de phosphore par l'intermédiaire de l'atome d'azote.

14. Procédé selon la revendication 13, caractérisé en ce que l'on utilise une diphosphine de formule (III) dans laquelle $R_1$ et $R_2$ représente un radical aryle et A est un groupe chiral.

15. Procédé selon l'une quelconque des revendications 11 à 14, caractérisé en ce que la diphosphine chirale est la tétramenthyldiphosphine, le bis(diphénylphosphinométhyl)-1,2 cyclobutane, le bis(diphénylphosphinométhyl)-4,5 diméthyl-2,2 dioxolane, le bis(diphénylphosphinométhyl)-1,2 acénaphtène, le bis(diphénylphosphino)-1,4 diméthoxy-2,3 butane, le bis[(N,N'-diphénylphosphino] bis[N,N'-(phényl-1 ethyl)] diaza-1,4 butane.

16. Procédé selon l'une quelconque des revendications 1 à 15, caractérisé en ce que la quantité de complexe du rhodium exprimée en nombre d'atomes-grammes de rhodium par mole d'aldéhyde diénique est comprise entre $1 \times 10^{-4}$ et $1 \times 10^{-1}$.

17. Procédé selon l'une quelconque des revendications 1 à 16, caractérisé en ce que la quantité de phosphine exprimée par le rapport du nombre d'atomes-grammes de phosphore au nombre d'atomes-grammes de rhodium est telle que ce rapport est compris entre 1 et 6.

18. Procédé selon l'une quelconque des revendications 1 à 17, caractérisé en ce que l'hydrogénation est conduite à une température comprise entre 0 est 150°C et sous une pression d'hydrogène comprise entre 0,1 et 100 bars.

**Patentansprüche**

1. Verfahren zur Herstellung von optisch aktivem Citronellal, dadurch gekennzeichnet, daß man Neral oder Geranial in Gegenwart eines Katalysators hydriert, der aus einem in dem Reaktionsmilieu löslichen Komplex besteht, gebildet aus einem Rhodiumderivat und einem chiralen Phosphin.

2. Verfahren gemäß Anspruch 1, dadurch gekennzeichnet, daß der Komplex des Rhodiums und des chiralen Phosphins frisch zubereitet wird.

3. Verfahren gemäß Anspruch 1, dadurch gekennzeichnet, daß der Komplex des Rhodiums und des chiralen Phosphins "in situ" aus einem Derivat des Rhodiums und einem chiralen Phosphin gebildet wird.

4. Verfahren gemäß Anspruch 3, dadurch gekennzeichnet, daß das Derivat des Rhodiums ein Salz einer Mineralsäure oder organischen Säure oder ein Komplex des Rhodiums mit einem achiralen Liganden ist.

5. Verfahren gemäß Anspruch 4, dadurch gekennzeichnet, daß das Derivat des Rhodiums das Rhodiumtrichlorid ist.

6. Verfahren gemäß Anspruch 4, dadurch gekennzeichnet, daß der Komplex ders Rhodiums die allgemeine Formel

$$[Rh\ X\ (L)_x]_2 \qquad (I)$$

hat, worin:

X ein Halogenatom bedeutet,

x eine ganze Zahl von 1 bis 4 ist,

L ein Mono- oder Diolefin bedeutet.

7. Verfahren gemäß Anspruch 6, dadurch gekennzeichnet, daß der Komplex des Rhodiums das $\mu,\mu'$-Dichlor-bis-(cyclooctadien-1,5-rhodium) ist.

8. Verfahren gemäß Anspruch 4, dadurch gekennzeichnet, daß der Komplex des Rhodiums die allgemeine Formel

$$Rh\ H\ (CO)\ (PR_3)_3 \qquad (II)$$

hat, worin R einen achiralen Alkyl-, Cycloalkyl- oder Arylrest mit 1 bis 10 Kohlenstoffatomen bedeutet.

9. Verfahren gemäß Anspruch 8, dadurch gekennzeichnet, daß der Komplex des Rhodiums die Formel $Rh\ H\ (CO)[P—(C_6H_5)_3]_3$ hat.

10. Verfahren gemäß Anspruch 4, dadurch gekennzeichnet, daß der Komplex des Rhodiums ein Rhodiumcarbonyl ist, genommen aus der Gruppe von Tetrarhodiumdodecarbonyl und von Hexarhodiumhexadecacarbonyl.

11. Verfahren gemäß einem der Ansprüche 1 bis 10, dadurch gekennzeichnet, daß das chirale Phosphin wenigstens ein chirales Kohlenstoffatom und/oder wenigstens ein chirales Phosphoratom aufweist.

12. Verfahren gemäß Anspruch 11, dadurch gekennzeichnet, daß das chirale Phosphin ein Diphosphin der allgemeinen Formel

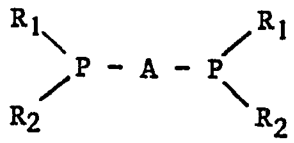

(III)

ist, worin:

$R_1$ und $R_2$, welche identisch oder verschieden sind, Kohlenwasserstoffreste mit 1 bis 15 Kohlenstoffatomen bedeuten,

A eine Valenzbindung oder einen zweiwertigen organischen Rest bedeutet, der gegebenenfalls durch eine oder mehrere inerte funktionelle Gruppen substituiert ist, wobei wenigstens einer der Reste $R_1$, $R_2$ und A chiral ist.

13. Verfahren gemäß Anspruch 12, dadurch gekennzeichnet, daß man ein chirales Diphosphin der Formel (III) verwendet, worin $R_1$ und $R_2$ Alkylreste mit 1 bis 10 Kohlenstoffatomen, Cycloalkylreste mit 4 bis 8 cyclischen Kohlenstoffatomen, Aryl- oder Alkylarylreste bedeuten und A darstellt:

a) einen linearen oder verzweigten Alkylenrest mit 1 bis 10 Kohlenstoffatomen, einen Cycloalkylenrest mit 3 bis 7 cyclischen Kohlenstoffatomen, die gegebenenfalls durch 1 bis 3 Alkylreste mit 1 bis 4 Kohlenstoffatomen substituiert ist, einen Arylenrest, einen zweiwertigen polycyclischen Rest, wobei diese Reste gegebenenfalls durch eine oder mehrere Alkoxygruppen mit 1 bis 4 Kohlenstoffatomen substituiert sein können;

b) eine zweiwertige heterocyclische Gruppe mit 1 oder 2 Heteroatomen aus der Gruppe von Sauerstoff oder Stickstoff;

c) eine Verkettung von einem oder mehreren zweiwertigen Alkylenresten und/oder Cycloalkylenresten und/oder heterocyclischen und/oder polycyclischen Resten, wie diejenigen die oben unter a) und b) definiert sind;

d) eine Verkettung von Alkylengruppen wie diejenigen die oben unter a) definiert sind und von tertiären Aminogruppen, die an die Phosphoratome über das Stickstoffatom direkt gebunden sein können.

14. Verfahren gemäß Anspruch 13, dadurch gekennzeichnet, daß man ein Diphosphin der Formel (III) verwendet, worin $R_1$ und $R_2$ einen Arylrest und A eine chirale Gruppe bedeuten.

15. Verfahren gemäß einem der Anspruch 11 bis 14, dadurch gekennzeichnet, daß das chirale Diphosphin das Tetramethyldiphosphin, das 1,2-Bis-(diphenylphosphinomethyl)-cyclobutan, das 4,5-Bis-(diphenylphosphinomethyl)-2,2-dimethyldioxolan, das 1,2-Bis-(diphenylphosphinomethyl)-acenaphthen, das 1,4-Bis-(diphenylphosphino)-2,3-dimethoxybutan, das 1,4-Bis-[N,N′-diphenylphosphino]-bis-[N,N′-(1-phenyläthyl)]-diaza-butan ist.

16. Verfahren gemäß einem der Ansprüche 1 bis 15, dadurch gekennzeichnet, daß die Menge an Komplex des Rhodiums ausgedrückt in Anzahl Grammatome Rhodium je Mol dienischer Aldehyd zwischen $1 \times 10^{-4}$ und $1 \times 10^{-1}$ beträgt.

17. Verfahren gemäß einem der Ansprüche 1 bis 16, dadurch gekennzeichnet, daß die Menge an Phosphin ausgedrückt durch das Verhältnis der Anzahl Grammatome Phosphor zur Anzahl Grammatome Rhodium derart ist, daß dieses Verhältnis zwischen 1 und 6 beträgt.

18. Verfahren gemäß einem der Ansprüche 1 bis 17, dadurch gekennzeichnet, daß die Hydrierung bei einer Temperatur zwischen 0 und 150°C und unter einem Wasserstoffdruck zwischen 0,1 und 100 bar durchgeführt wird.

**Claims**

1. Process for the preparation of optically active citronellal, characterised in that neral or geranial is hydrogenated in the presence of a catalyst consisting of a complex which is soluble in the reaction medium and which is formed from a rhodium derivative and a chiral phosphine.

2. Process according to Claim 1, characterised in that the complex of rhodium and the chiral phosphine is prepared for immediate use.

3. Process according to Claim 1, characterised in that the complex of rhodium and the chiral phosphine is formed "in situ" from a rhodium derivative and a chiral phosphine.

4. Process according to Claim 3, characterised in that the rhodium derivative is a salt of a mineral or organic acid or a rhodium complex with an achiral ligand.

5. Process according to Claim 4, characterised in that the rhodium derivative is rhodium trichloride.

6. Process according to Claim 4, characterised in that the rhodium complex has the general formula:

$$[RhX(L)_x]_2 \qquad (I)$$

in which X represents a halogen atom, x is an integer from 1 to 4 and L represents a monoolefine or diolefine.

7. Process according to Claim 6, characterised in that the rhodium complex is $\mu,\mu'$-dichloro-bis-(cycloocta-1,5-diene-rhodium).

8. Process according to Claim 4, characterised in that the rhodium complex has the general formula:

$$RhH(CO)(PR_3)_3 \qquad (II)$$

in which R represents an achiral alkyl, cycloalkyl or aryl radical having from 1 to 10 carbon atoms.

9. Process according to Claim 8, characterised in that the rhodium complex has the formula

$$RhH(CO)[P{-\!\!-}(C_6H_5)_3]_3.$$

10. Process according to Claim 4, characterised in that the rhodium complex is a rhodium carbonyl taken from the group comprising tetrarhodium dodecacarbonyl and hexarhodium hexadecacarbonyl.

11. Process according to any one of Claims 1 to 10, characterised in that the chiral phosphine contains at least one chiral carbon atom and/or at least one chiral phosphorus atom.

12. Process according to Claim 11, characterised in that the chiral phosphine is a diphosphine of the general formula:

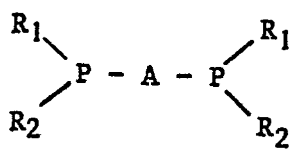

(III)

in which $R_1$ and $R_2$, which are identical or different, represent hydrocarbon radicals having from 1 to 15 carbon atoms and A represents a valence bond or a divalent organic radical which is optionally substituted by one or more inert functional groups, at least one of the radicals $R_1$, $R_2$ and A being chiral.

13. Process according to Claim 12, characterised in that a chiral diphosphine of the formula (III) is used in which $R_1$ and $R_2$ represent alkyl radicals having from 1 to 10 carbon atoms, cycloalkyl radicals having from 4 to 8 ring carbon atoms, or aryl or alkylaryl radicals, and A symbolises:

a) A linear or branched alkylene radical having from 1 to 10 carbon atoms, a cycloalkylene radical having from 3 to 7 ring carbon atoms which is optionally substituted by 1 to 3 alkyl radicals having from 1 to 4 carbon atoms, an arylene radical or a divalent polycyclic radical, it being possible for the said radicals to be substituted by one or more alkoxy groups having from 1 to 4 carbon atoms;

b) a divalent heterocyclic group having 1 or 2 heteroatoms from the group comprising oxygen and nitrogen;

c) an arrangement of 1 or more alkylene and/or cycloalkylene and/or heterocyclic and/or divalent polycyclic radicals such as those defined above under a) and b); or

d) an arrangement of alkylene groups, such as those defined above under a), and of tertiary amino groups which can be directly bonded to the phosphorus atoms via the nitrogen atom.

14. Process according to Claim 13, characterised in that a diphosphine of the formula (III) is used in which $R_1$ and $R_2$ represent an aryl radical and A is a chiral group.

15. Process according to any one of Claims 11 to 14, characterised in that the chiral diphosphine is tetramenthyldiphosphine, 1,2-bis-(diphenylphosphinomethyl)cyclobutane, 4,5-bis-(diphenyl-phosphinomethyl)-2,2-dimethyldioxolane, 1,2-bis-(diphenylphosphinomethyl)acenaphthene, 1,4-bis(diphenylphosphino)-2,3-dimethoxybutane or [N,N'-bis-(diphenylphosphino)]-[N,N'-bis-(1-phenyl-ethyl)]-1,4-diazabutane.

16. Process according to any one of Claims 1 to 15, characterised in that the amount of rhodium complex, expressed as the number of gram atoms of rhodium per mol of dienaldehyde, is between $1 \times 10^{-4}$ and $1 \times 10^{-1}$.

17. Process according to any one of Claims 1 to 16, characterised in that the amount of phosphine, expressed as the ratio of the number of gram atoms of phosphorus to the number of gram atoms of rhodium, is such that this ratio is between 1 and 6.

18. Process according to any one of Claims 1 to 17, characterised in that the hydrogenation is carried out at a temperature between 0 and 150°C and under a hydrogen pressure between 0.1 and 100 bars.